# EUROPEAN PATENT APPLICATION

(11) **EP 4 806 490 A1**
(43) Date of publication of application: **16.09.2026**
(21) Application number: 23958318.0
(22) Date of filing: 10.11.2023
(51) Int. Cl.: A61M 25/09

(54) **MEDICAL DEVICE**

(71) Applicant: ASAHI INTECC CO., LTD., Seto-shi, Aichi, 489-0071 (JP)
(72) Inventor: FUKUSHIMA Kensei, Seto-shi, Aichi 489-0071 (JP); NISHIGISHI Makoto, Seto-shi, Aichi 489-0071 (JP); KIDO Yusuke, Seto-shi, Aichi 489-0071 (JP)
(74) Representative: Prinz & Partner mbB
(86) International application number: PCT/JP2023/040502
(87) International publication number: WO 2025/099917

(57) **Abstract**

A medical device includes a distal tip, an outer layer coil, and a coating. The outer layer coil is connected to the distal tip. The coating covers an outer peripheral surface of the distal tip and an outer peripheral surface of the outer layer coil in a region from a distal end of the distal tip to a predetermined axial position. The axial position is located on a distal side relative to a proximal end of the outer layer coil.

## Description

### TECHNICAL FIELD

The technology disclosed herein relates to a medical device.

### BACKGROUND ART

Methods using catheters are widely performed to treat or examine, for example, a stenosed portion or an occluded portion (hereinafter referred to as a "lesion portion") in a blood vessel. A guidewire is used to guide the catheter to the lesion portion in the blood vessel. The guidewire has a distal tip and an outer layer coil connected to the distal tip.

A guidewire in which the entirety of an outer layer coil is covered with a coating is known (see, for example, Patent Literature 1).

### CITATION LIST

### Patent Literature

Patent Literature 1: US Patent Application Publication No. 2004/0039304

### SUMMARY OF INVENTION

### TECHNICAL PROBLEM

Guidewires may require specific surface characteristics. For example, a guidewire may require surface characteristics capable of achieving both an improvement in passability and a reduction in the risk of vascular perforation. Conventional guidewires face difficulties in achieving desired surface characteristics. Such a problem is not limited to guidewires but is a problem common to medical devices.

This specification discloses a technology capable of solving the above-described problem.

### SOLUTION TO PROBLEM

(1) A medical device (100) disclosed herein includes a distal tip (40), an outer layer coil (20), and a coating (60). The outer layer coil (20) is connected to the distal tip (40). The coating (60) covers an outer peripheral surface of the distal tip (40) and an outer peripheral surface of the outer layer coil (20) in a region (R1) from a distal end (41) of the distal tip (40) to a predetermined axial position (P1). The axial position (P1) is located on a distal side relative to a proximal end (22) of the outer layer coil (20).
   In this medical device, the surface of the outer layer coil is covered with the coating in the region on the distal side relative to the predetermined axial position, and the surface of the outer layer coil is not covered with the coating in the region on the proximal side relative to the predetermined axial position. Therefore, desired surface characteristics can be imparted to each region along the axial direction of the medical device. For example, in the region on the distal side relative to the predetermined axial position, since the surface of the outer layer coil is covered with the coating, lubricity of the medical device is improved and passability is improved. In the region on the proximal side relative to the predetermined axial position, since the surface of the outer layer coil is not covered with the coating, the lubricity of the medical device is suppressed, and the risk of perforation of a blood vessel or the like is reduced. As a result, in the medical device, it is possible to realize both improvement in passability and reduction in risk of perforation of a blood vessel or the like.
(2) In the medical device described above, the coating may be made of urethane resin. According to this aspect, the lubricity of the medical device can be effectively improved in the region where the surface of the outer layer coil is covered with the coating, and the passability of the medical device can be effectively improved.
(3) The medical device described above may further include a core shaft disposed inside the outer layer coil, and a joining portion joining the core shaft and the outer layer coil, and the axial position may be located on a proximal side relative to the joining portion. According to this aspect, a size of the region where the surface of the outer layer coil is covered with the coating can be secured to a certain extent or more, and the passability of the medical device can be effectively improved.
(4) The medical device described above may further include an inner layer coil connected to the distal tip and disposed inside the outer layer coil, and the axial position may be located on a proximal side relative to a proximal end of the inner layer coil. According to this aspect, the size of the region where the surface of the outer layer coil is covered with the coating can be secured to a certain extent or more, and the passability of the medical device can be effectively improved.
(5) In the medical device described above, the coating may be a first coating, and the medical device may further include a second coating covering an outer circumference of the outer layer coil. According to this aspect, the second coating can improve the lubricity of the medical device, and can improve the passability of the medical device.
(6) In the medical device described above, the second coating may be hydrophilic. According to this aspect, the presence of the second coating can effectively improve the lubricity of the medical device, and can effectively improve the passability of the medical device.
(7) In the medical device described above, the second coating may cover the first coating in a region on the distal side relative to the axial position. According to this aspect, in the region on the distal side relative to the predetermined axial position, the presence of the first coating and the second coating can effectively improve the lubricity of the medical device, and can effectively improve the passability of the medical device.
(8) In the medical device described above, lubricity in the region from the distal end of the distal tip to the axial position may be higher than lubricity in a region from the axial position to the proximal end of the outer layer coil. According to this aspect, it is possible to increase the lubricity of the region from the distal end of the distal tip to the predetermined axial position in the medical device to improve the passability of the medical device, while suppressing the lubricity of the region on the proximal side relative to the region to reduce the risk of perforation of a blood vessel or the like by the medical device.

The technology disclosed herein can be realized in various aspects, for example, in aspects such as a medical device and a method for manufacturing the same.

### BRIEF DESCRIPTION OF DRAWINGS

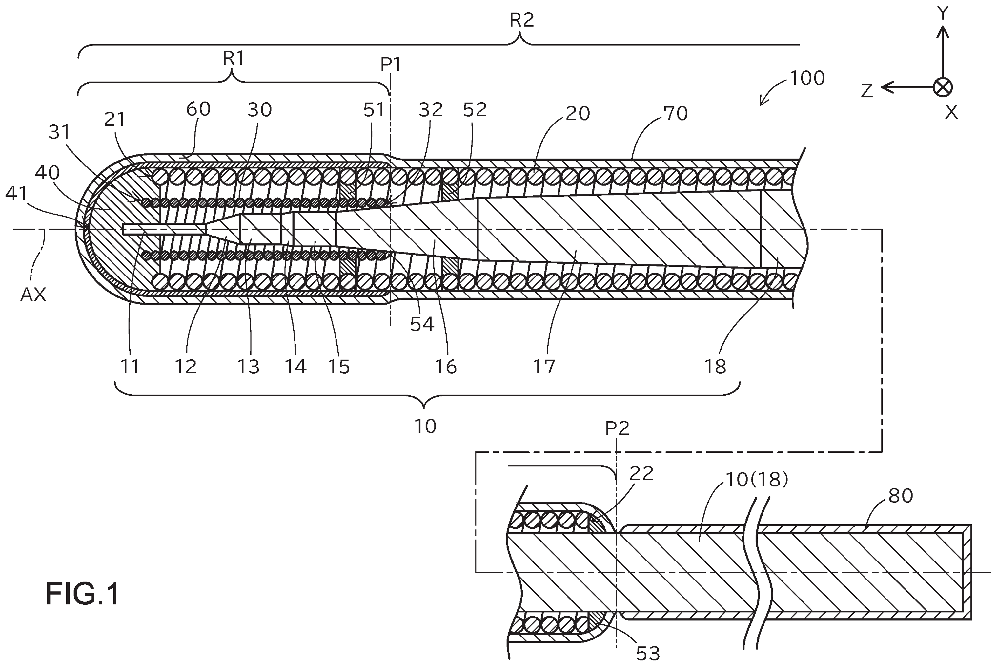

FIG. 1 is an explanatory diagram schematically illustrating a configuration of a guidewire according to an embodiment.

### EMBODIMENTS OF THE INVENTION

### A. Embodiment

### (Configuration of Guidewire 100)

FIG. 1 is an explanatory diagram schematically illustrating a configuration of a guidewire 100 according to an embodiment. FIG. 1 shows XYZ axes orthogonal to one another for specifying directions, and shows a longitudinal section (YZ section) of the guidewire 100. Along a direction parallel to a central axis AX of the guidewire 100 (hereinafter referred to as an "axial direction"), a Z-axis positive direction side is a distal side (distal end side) to be inserted into a body, and a Z-axis negative direction side is a proximal side (proximal end side) to be operated by a physician. FIG. 1 shows a state in which the guidewire 100 has a linear shape substantially parallel to the Z-axis direction as a whole. The guidewire 100 has flexibility to an extent that allows bending. In this specification, regarding the guidewire 100 and each component thereof, an end on the distal side is referred to as a "distal end," the distal end and its vicinity are referred to as a "distal end portion," an end on the proximal side is referred to as a "proximal end," and the proximal end and its vicinity are referred to as a "proximal end portion."

The guidewire 100 is a medical device. The guidewire 100 is inserted into a blood vessel, for example, to guide another medical device (not shown) such as a catheter to a lesion portion in the blood vessel. The guidewire 100 includes a core shaft 10, an outer layer coil 20, an inner layer coil 30, a distal tip 40, a first intermediate joining portion 51, a second intermediate joining portion 52, a first proximal side joining portion 53, a second proximal side joining portion 54, a first coating 60, a second coating 70, and a third coating 80.

The core shaft 10 is an elongated member. A central axis of the core shaft 10 substantially coincides with the central axis AX of the guidewire 100. The core shaft 10 has a first portion 11, a second portion 12, a third portion 13, a fourth portion 14, a fifth portion 15, a sixth portion 16, a seventh portion 17, and an eighth portion 18. The first portion 11, the second portion 12, the third portion 13, the fourth portion 14, the fifth portion 15, the sixth portion 16, the seventh portion 17, and the eighth portion 18 are arranged in this order from the distal end toward the proximal side.

In the present embodiment, the first portion 11, the third portion 13, the fifth portion 15, and the eighth portion 18 of the core shaft 10 have a constant cross-sectional shape (XY section) at each position along the axial direction. An area of the cross section of the third portion 13 is larger than an area of the cross section of the first portion 11. An area of the cross section of the fifth portion 15 is larger than the area of the cross section of the third portion 13. An area of the cross section of the eighth portion 18 is larger than the area of the cross section of the fifth portion 15. The second portion 12, the fourth portion 14, the sixth portion 16, and the seventh portion 17 of the core shaft 10 smoothly connect cross-sectional shapes of other adjacent portions along the axial direction. The second portion 12, the fourth portion 14, the sixth portion 16, and the seventh portion 17 are tapered portions in which the area of the cross section gradually increases from the distal side toward the proximal side.

The core shaft 10 having such a shape can be manufactured, for example, by performing press working on a precursor having a constant cross section along the axial direction at a press rate corresponding to the shape of each portion of the core shaft 10.

Examples of a material for forming the core shaft 10 include metal materials, and more specifically, stainless steel (SUS302, SUS304, SUS316, etc.), nickel-titanium alloys, piano wire, nickel-chromium alloys, cobalt alloys, tungsten, and the like.

The outer layer coil 20 is a member formed in a hollow cylindrical shape by spirally winding a wire. The outer layer coil 20 is, for example, a densely wound coil. The outer layer coil 20 is disposed so as to surround an outer circumference of the distal end portion of the core shaft 10. In the axial direction, a position of a distal end 21 of the outer layer coil 20 is substantially the same as a position of the distal end of the core shaft 10.

The inner layer coil 30 is a member formed in a hollow cylindrical shape by spirally winding a wire. The inner layer coil 30 is, for example, a densely wound coil. The inner layer coil 30 is disposed so as to surround the outer circumference of the distal end portion of the core shaft 10 in a space inside the outer layer coil 20. In the axial direction, a position of a distal end 31 of the inner layer coil 30 is substantially the same as the position of the distal end of the core shaft 10, and a position of a proximal end 32 of the inner layer coil 30 is on the distal side relative to a position of a proximal end 22 of the outer layer coil 20.

Outer diameters and inner diameters of the outer layer coil 20 and the inner layer coil 30 may be constant along the axial direction, or may change along the axial direction.

Examples of materials for forming the outer layer coil 20 and the inner layer coil 30 include metal materials, more specifically, radiolucent alloys such as stainless steel (SUS302, SUS304, SUS316, etc.), nickel-titanium alloys, piano wire, nickel-chromium alloys, or cobalt alloys, and radiopaque alloys such as gold, platinum, tungsten, or alloys containing these elements (e.g., platinum-nickel alloys).

The distal tip 40 joins the distal end portion of the core shaft 10 and distal end portions of the outer layer coil 20 and the inner layer coil 30. That is, the outer layer coil 20 and the inner layer coil 30 are connected to the distal tip 40. An outer peripheral surface on the distal side of the distal tip 40 is a smooth surface (e.g., a substantially hemispherical surface). The first proximal side joining portion 53 joins the core shaft 10 and a proximal end portion of the outer layer coil 20. The second proximal side joining portion 54 joins the core shaft 10 and a proximal end portion of the inner layer coil 30. The first intermediate joining portion 51 joins the core shaft 10 and an intermediate portion of the outer layer coil 20. The intermediate portion of the outer layer coil 20 is a portion excluding the distal end portion and the proximal end portion of the outer layer coil 20 (the same applies hereinafter). The first intermediate joining portion 51 joins the core shaft 10 and an intermediate portion of the inner layer coil 30. The first intermediate joining portion 51 is an example of a joining portion. The second intermediate joining portion 52 is located on the proximal side relative to the first intermediate joining portion 51, and joins the core shaft 10 and the intermediate portion of the outer layer coil 20. Examples of materials for forming the distal tip 40 and the respective joining portions 51, 52, 53, 54 include metal solders such as silver solder, gold solder, zinc, Sn-Ag alloys, and Au-Sn alloys, and adhesives such as epoxy adhesives.

The first coating 60 covers the outer peripheral surface of the distal tip 40 and the outer peripheral surface of the outer layer coil 20 in a first region R1 from a distal end 41 of the distal tip 40 to a predetermined position in the axial direction (hereinafter referred to as a "first axial position P1") in the guidewire 100. Examples of a material for forming the first coating 60 include urethane resin.

The second coating 70 covers the outer peripheral surface of the outer layer coil 20 in a second region R2 from the distal end 41 of the distal tip 40 to a predetermined position in the axial direction (hereinafter referred to as a "second axial position P2") in the guidewire 100. More specifically, in the second region R2 excluding the first region R1, the second coating 70 covers the outer peripheral surface of the outer layer coil 20, and in the first region R1, the second coating 70 covers the first coating 60. Such a configuration can be realized, for example, by forming the first coating 60 in the first region R1 and then forming the second coating 70 in the second region R2 encompassing the first region R1. The second coating 70 is, for example, a hydrophilic coating. Examples of a material for forming the second coating 70 include polyvinylpyrrolidone, polyacrylic acid, polyacrylamide, polyvinyl alcohol, maleic anhydride copolymer, hyaluronic acid, and the like.

The third coating 80 covers an outer peripheral surface of the core shaft 10 in a region on the proximal side relative to the proximal end 22 of the outer layer coil 20 in the guidewire 100. Examples of a material for forming the third coating 80 include polytetrafluoroethylene (PTFE).

### (Effects of Present Embodiment)

As described above, the guidewire 100 of the present embodiment includes the distal tip 40, the outer layer coil 20, and the first coating 60. The outer layer coil 20 is connected to the distal tip 40. The first coating 60 covers the outer peripheral surface of the distal tip 40 and the outer peripheral surface of the outer layer coil 20 in the first region R1 from the distal end 41 of the distal tip 40 to the first axial position P1. The first axial position P1 is located on the distal side relative to the proximal end 22 of the outer layer coil 20. Thus, in the guidewire 100 of the present embodiment, the surface of the outer layer coil 20 is covered with the first coating 60 in the first region R1 on the distal side relative to the first axial position P1, and the surface of the outer layer coil 20 is not covered with the first coating 60 in the region on the proximal side relative to the first axial position P1. Therefore, according to the guidewire 100 of the present embodiment, desired surface characteristics can be imparted to each region along the axial direction. For example, in the first region R1 on the distal side relative to the first axial position P1, since the surface of the outer layer coil 20 is covered with the first coating 60, the lubricity of the guidewire 100 is improved and the passability is improved. In the region on the proximal side relative to the first axial position P1, since the surface of the outer layer coil 20 is not covered with the first coating 60, the lubricity of the guidewire 100 is suppressed and the risk of vascular perforation is reduced. As a result, in the guidewire 100, it is possible to realize both improvement in passability and reduction in risk of vascular perforation.

The first coating 60 may be made of urethane resin. In this way, the lubricity of the guidewire 100 can be effectively improved in the first region R1 where the surface of the outer layer coil 20 is covered with the first coating 60, and the passability of the guidewire 100 can be effectively improved.

The guidewire 100 may further include the core shaft 10 disposed inside the outer layer coil 20, and the first intermediate joining portion 51 joining the core shaft 10 and the outer layer coil 20, and the first axial position P1 may be located on the proximal side relative to the first intermediate joining portion 51. In this way, the size of the first region R1 where the surface of the outer layer coil 20 is covered with the first coating 60 can be secured to a certain extent or more, and the passability of the guidewire 100 can be effectively improved.

The guidewire 100 may further include the inner layer coil 30 connected to the distal tip 40 and disposed inside the outer layer coil 20, and the first axial position P1 may be located on the proximal side relative to the proximal end 32 of the inner layer coil 30. In this way, the size of the first region R1 where the surface of the outer layer coil 20 is covered with the first coating 60 can be secured to a certain extent or more, and the passability of the guidewire 100 can be effectively improved.

The guidewire 100 may further include the second coating 70 covering the outer circumference of the outer layer coil 20. In this way, the presence of the second coating 70 makes it possible to improve the lubricity of the guidewire 100, and improve the passability of the guidewire 100.

The second coating 70 may be hydrophilic. In this way, the presence of the second coating 70 makes it possible to effectively improve the lubricity of the guidewire 100, and effectively improve the passability of the guidewire 100.

The second coating 70 may cover the first coating 60 in a region on the distal side relative to the first axial position P1. In this way, in the region on the distal side relative to the first axial position P1, the presence of the first coating 60 and the second coating 70 makes it possible to effectively improve the lubricity of the guidewire 100, and effectively improve the passability of the guidewire 100.

The lubricity in the first region R1 from the distal end 41 of the distal tip 40 to the first axial position P1 in the guidewire 100 may be higher than the lubricity in the region from the first axial position P1 to the proximal end 22 of the outer layer coil 20. In this way, it is possible to increase the lubricity of the first region R1 in the guidewire 100 to improve the passability of the guidewire 100, while suppressing the lubricity of the region on the proximal side relative to the first region R1 to reduce the risk of vascular perforation by the guidewire 100.

### B. Modifications

The technology disclosed herein is not limited to the above-described embodiment, and can be modified into various modifications without departing from the gist thereof. For example, the following modifications are also possible.

The configuration of the guidewire 100 in the above embodiment is merely an example, and various modifications are possible. For example, the proximal end (first axial position P1) of the region where the surface of the outer layer coil 20 is covered with the first coating 60 can be arbitrarily modified as long as it is located on the distal side relative to the proximal end 22 of the outer layer coil 20.

The proximal end of the region where the surface of the outer layer coil 20 is covered with the second coating 70 can be arbitrarily modified as long as it is located on the proximal side relative to the first axial position P1.

The guidewire 100 may not include the inner layer coil 30, or the guidewire 100 may include another coil other than the outer layer coil 20 and the inner layer coil 30.

The guidewire 100 may not include the second coating 70 and/or the third coating 80, or the guidewire 100 may include another coating other than the first coating 60, the second coating 70, and the third coating 80.

The positions of the respective joining portions 51, 52, 53, 54 included in the guidewire 100 are merely examples, and various modifications are possible. At least one of the respective joining portions 51, 52, 53, 54 may be omitted.

The technology disclosed herein is applicable not only to the guidewire 100 but also to medical devices in general.

## Claims

1. A medical device (100) comprising:
a distal tip (40);
an outer layer coil (20) connected to the distal tip (40); and
a coating (60) covering an outer peripheral surface of the distal tip (40) and an outer peripheral surface of the outer layer coil (20) in a region (R1) from a distal end (41) of the distal tip (40) to a predetermined axial position (P1), wherein
the axial position (P1) is located on a distal side relative to a proximal end (22) of the outer layer coil (20).

2. The medical device (100) according to claim 1, wherein the coating (60) is made of urethane resin.

3. The medical device (100) according to claim 1 or 2, further comprising:
a core shaft (10) disposed inside the outer layer coil (20); and
a joining portion (51) joining the core shaft (10) and the outer layer coil (20), wherein
the axial position (P1) is located on a proximal side relative to the joining portion (51).

4. The medical device (100) according to any one of claims 1 to 3, further comprising an inner layer coil (30) connected to the distal tip (40) and disposed inside the outer layer coil (20), wherein
the axial position (P1) is located on a proximal side relative to a proximal end (32) of the inner layer coil (30).

5. The medical device (100) according to any one of claims 1 to 4, wherein
the coating (60) is a first coating (60), and
the medical device (100) further comprises a second coating (70) covering an outer circumference of the outer layer coil (20).

6. The medical device (100) according to claim 5, wherein the second coating (70) is hydrophilic.

7. The medical device (100) according to claim 5 or 6, wherein the second coating (70) covers the first coating (60) in a region on a distal side relative to the axial position (P1).

8. The medical device (100) according to claim 7, wherein lubricity in the region (R1) from the distal end (41) of the distal tip (40) to the axial position (P1) is higher than lubricity in a region from the axial position (P1) to the proximal end (22) of the outer layer coil (20).
